# EUROPÄISCHE PATENTSCHRIFT

(11) **EP 0 931 112 B1**
(45) Veröffentlichungstag und Bekanntmachung des Hinweises auf die Patenterteilung: **08.01.2003**
(21) Anmeldenummer: 97923992.8
(22) Anmeldetag: 23.05.1997
(51) Int. Cl.: C09C 1/00, C01G 23/00, C09D 7/12, C09D 11/00, A61K 7/00, C08K 3/00, C04B 33/14, C03C 4/02

(54) **METALLOXIDBESCHICHTETE TITANDIOXIDPLÄTTCHEN**
METAL OXIDE COATED TITANIUM DIOXIDE LAMELLAS
PAILLETTES DE DIOXYDE DE TITANE REVETUES D'OXYDE METALLIQUE

(43) Veröffentlichungstag der Anmeldung: 28.07.1999
(73) Patentinhaber: MERCK PATENT GmbH, 64271 Darmstadt (DE)
(72) Erfinder: ANDES, Stephanie, D-63477 Maintal 3 (DE); BAUER, Gerd, D-63801 Kleinostheim (DE); BRENNER, Günter, D-64347 Griesheim (DE); BRÜCKNER, Dieter, D-64289 Darmstadt (DE); SCHMELZ, Michael, D-65830 Kriftel (DE); HEYLAND, Andrea, D-64385 Ober-Kainsbach (DE); KUNTZ, Matthias, D-64342 Seeheim (DE); OSTERRIED, Karl, D-64807 Dieburg (DE); PFAFF, Gerhard, D-64839 Münster (DE)
(86) Internationale Anmeldenummer: EP9702650
(87) Internationale Veröffentlichungsnummer: WO98053010

(56) Entgegenhaltungen:
- EP-A- 0 045 851
- EP-A- 0 211 351
- EP-A- 0 307 747
- DE-A- 1 467 468
- FR-A- 1 273 230
- FR-A- 2 198 984
- JP-A- 5 888 121
- US-A- 2 941 895
- DATABASE WPI Week 8706 Derwent Publications Ltd., London, GB; AN 87-039671 XP002037924 & JP 61 295 234 A (SUMITUMO CHEM. IND.) , 26.Dezember 1986

## Beschreibung

Die Erfindung betrifft sehr dünne Perlglanzpigmente auf der Basis von metalloxidbeschichtetem bzw. metalloxidhydratbeschichtetem plättchenförmigem Titandioxid.

Perlglanzpigmente auf Glimmerbasis, die auf einer Titandioxidschicht weitere Metalloxidschichten besitzen, sind bekannt. In US 3 087 828 und 3 087 829 werden als farblose Oxide für eine zweite Metalloxidschicht Aluminiumoxid, Zirkoniumoxid, Zinkoxid und Zinnoxid und als Oxide, die eine Eigenfärbung aufweisen, Eisenoxid, Nickeloxid, Kobaltoxid, Kupferoxid und Chromoxid genannt. Die Abscheidung eines zweiten Metalloxides auf der Titandioxidhydratschicht führt zu einer deutlichen Stabilisierung bezüglich der Lichtempfindlichkeit dieser Schicht. Insbesondere eisenoxidhaltige Titandioxidpigmente werden seit vielen Jahren mit Erfolg angewendet.

In US 30 87 828 wird beschrieben, daß durch Abscheiden einer Fe₂O₃-Schicht auf eine TiO₂-Schicht goldfarbene Glimmerpigmente erhalten werden, die beim Glühen einen rötlichen Farbton annehmen.

In US 3 874 890 wird ein Verfahren zur Herstellung von goldfarbenen Perlglanzpigmenten beschrieben, bei dem ein mit TiO₂ und/oder ZrO₂ beschichtetes Glimmerpigment zunächst mit Eisen(II)-hydroxid beschichtet wird, das dann zu Fe₂O₃ oxidiert wird.

US 4 744 832 beschreibt ein Perlglanzpigment auf Basis von mit Metalloxiden überzogenen plättchenförmigen Substraten, insbesondere Glimmer, wobei die Metalloxidschicht sowohl Titan als auch Eisen enthält und das Pigment einen Mehrschichtaufbau besitzt, wobei auf eine erste Schicht von TiO₂ in der Rutilform eine Schicht von Pseudobrookit und eine Eisenoxidschicht folgt.

Glimmerpigmente werden in großem Umfang in der Druck- und Lackindustrie, in der Kosmetik und in der Kunststoffverarbeitung verwendet. Sie zeichnen sich durch Interferenzfarben und einen hohen Glanz aus. Für die Ausbildung extrem dünner Schichten sind aber Glimmerpigmente nicht geeignet, weil Glimmer als Substrat für die Metalloxidschichten des Pigmentes bereits eine Dicke von 200 bis 1200 nm aufweist. Nachteilig ist weiterhin, daß die Dicke der Glimmerplättchen innerhalb einer bestimmten Fraktion, die durch die Plättchengröße bestimmt wird, teilweise deutlich um einen Mittelwert schwankt. Außerdem handelt es sich bei Glimmer um ein natürlich vorkommendes Mineral, das durch Fremdionen verunreinigt ist. Desweiteren sind technisch sehr aufwendige und zeitraubende Aufbereitungsschritte notwendig. Hierzu zählen vor allem Mahlen und Klassieren.

Auf dicken Glimmerplättchen basierende, mit Metalloxiden umhüllte Perlglanzpigmente haben aufgrund der Dicke des Randes einen deutlichen Streuanteil, speziell bei feineren Korngrößenverteilungen unter 20 µm.

Als Ersatz für Glimmer sind dünne Glasplättchen vorgeschlagen worden, die durch Walzen einer Glasschmelze mit nachfolgendem Mahlen erhalten werden. Interferenzpigmente auf der Basis derartiger Materialien weisen zwar Farbeffekte auf, die denen herkömmlicher, auf Glimmer basierender Pigmente überlegen sind. Nachteilig ist jedoch, daß die Glasplättchen eine sehr große mittlere Dicke von etwa 10-15 µm und eine sehr breite Dickenverteilung (typischerweise zwischen 4 und 20 µm) aufweisen, während die Dicke von Interferenzpigmenten typischerweise nicht größer als 3 µm ist.

In EP 0,384,596 wird ein Verfahren beschrieben, bei dem hydratisiertes Alkalisilikat bei Temperaturen von 480-500 °C mit einem Luftstrahl beaufschlagt wird, wobei sich Blasen mit dünnen Wandstärken bilden; die Blasen werden anschließend zerkleinert und man erhält plättchenförmige Alkalisilikatsubstrate mit einer Dicke von weniger als 3 µm. Das Verfahren ist jedoch aufwendig und die Dickenverteilung der erhaltenen Plättchen ist relativ breit.

In DE 11 36 042 ist ein kontinuierliches Bandverfahren zur Herstellung plättchen- oder flitterartiger Oxide oder Oxidhydrate von Metallen der IV. und V. Gruppe sowie der Eisen-Gruppe des Periodensystems beschrieben. Dabei wird auf ein kontinuierliches Band ggf. zunächst eine Trennschicht aus z.B. Silikonlack aufgebracht, um das spätere Ablösen der Metalloxidschicht zu erleichtern. Anschließend wird ein Flüssigkeitsfilm aus einer Lösung einer hydrolysierbaren Verbindung des in das gewünschte Oxid umzuwandelnden Metalls aufgebracht, der Film wird getrocknet und anschließend mit einer Rüttelvorrichtung abgelöst. Die Schichtdicke der erhaltenen Plättchen wird mit 0,2 bis 2 µm angegeben, ohne hierfür konkrete Beispiele zu nennen.

In EP 0,240,952 und EP 0,236,952 ist ein kontinuierliches Bandverfahren zur Herstellung verschiedener plättchenförmiger Materialien, darunter auch Siliciumdioxid, Aluminiumoxid und Titandioxid vorgeschlagen worden. Dabei wird über ein Rollensystem auf ein glattes Band ein dünner flüssiger Film definierter Dickes eines Precursors des plättchenförmigen Materials aufgebracht; der Film wird getrocknet und von dem Band abgelöst, wobei sich plättchenförmige Teilchen bilden. Die Teilchen werden anschließend gegebenenfalls geglüht, gemahlen und klassiert.

Die Dicke der nach dem in EP 0 240 952 beschriebenen Verfahren erhaltenen Plättchen ist relativ gut definiert, da der Film z.B. auf das kontinuierliche Band über ein Rollensystem sehr gleichmäßig aufgebracht wird. Die Schichtdicke der Plättchen wird in den Beispielen mit 0,3 bis 3,0 µm angegeben. Gemäß Beispiel 1 wird eine erste Rolle mit dem verwendeten Precursor benetzt, indem man diese Rolle teilweise in einen mit dem Precursor befüllten Vorratsbehälter eintaucht. Der Film wird von dieser Rolle auf eine zweite, gleichsinnig rotierende Rolle übertragen, die mit der ersten in sehr engem Kontakt steht. Schließlich wird der Film von der zweiten Rolle auf das kontinuierliche Band abgerollt.

Nachteilig sind jedoch die Verwendung sehr teurer Precursormaterialien sowie insbesondere die erhöhten Anforderungen an die Arbeitsplatzsicherheit, die beim Einsatz metallorganischer Verbindungen gestellt werden müssen. Die vollständige chemische Umwandlung des Precursors in das gewünschte Schichtmaterial macht in der Regel eine starke Erhitzung des Filmes und des Bandmaterials erforderlich. Neben der dabei auftretenden erheblichen thermischen Belastung des Bandmaterials, wirken sich auch der hohe Energieaufwand und die Einschränkung der Prozeßgeschwindigkeit sehr nachteilig auf die Wirtschaftlichkeit des Verfahrens aus.

WO 93/08 237 beschreibt plättchenförmige Pigmente, bestehend aus einer plättchenförmigen Matrix aus Siliciumdioxid, die lösliche oder nichtlösliche Farbmittel enthalten kann und die mit einer oder mehreren reflektierenden Schichten aus Metalloxiden oder Metallen belegt ist. Die plättchenförmige Matrix wird durch Verfestigung von Wasserglas auf einem endlosen Band hergestellt.

In DE 1 273 098 wird die Herstellung eines Perlmuttpigmentes durch Aufdampfen von ZnS-, MgF2-, ZnO-, CaF₂- und TiO₂-Filmen auf ein endloses Band beschrieben. Dieses Verfahren ist aber ebenso, wie das in US 4 879 140 beschriebene Verfahren, bei dem plättchenförmige Pigmente mit Si- und SiO₂-Schichten durch Plasmaabscheidung aus SiH₄ und SiCl₄ erhalten werden, mit einem sehr hohen apparativen Aufwand verbunden.

JP-A-61-295234 beschreibt ein plättchenförmiges Titandioxidpigment, welches mit Metalloxiden beschichtet ist. Die plättchenförmigen Titandioxidpartikel können hergestellt werden, indem auf glatten Oberflächen titanhaltige Lösungen aufgebracht, getrocknet und anschließend in plättchenförmige Partikel aufgebrochen werden. Nach der Trocknung und ggf. Mahlung werden diese mit Metalloxiden beschichtet.

Trotz zahlreicher Versuche konnte bisher noch kein wirtschaftliches Verfahren zur Herstellung sehr dünner plättchenförmiger Titandioxidpigmente mit einer Schichtdicke von kleiner als 500 nm entwickelt werden.

Aufgabe der Erfindung ist es, hochglänzende titandioxidhaltige Perlglanzpigmente mit einer Schichtdicke von kleiner als 500 nm und einer Schichtdickentoleranz von weniger als 10 % bereitzustellen.

Diese Aufgabe wird gemäß der Erfindung gelöst durch ein Perlglanzpigment mit einem Mehrschichtaufbau, wobei auf einen Kern aus plättchenförmigem Titandioxid eine Schicht aus einem anderen Metalloxid oder Metalloxidhydrat folgt, erhältlich durch Verfestigung einer wäßrigen Lösung einer thermisch hydrolysierbaren Titanverbindung auf einem endlosen Band, Ablösung der entstandenen Schicht, Beschichtung der erhaltenen Titandioxidplättchen ohne Zwischentrocknung im Naßverfahren mit einem anderen Metalloxid oder Metalloxidhydrat, Abtrennung, Trocknung und gegebenenfalls Kalzination des erhaltenen Materials.

Das andere Metalloxid oder Metalloxidhydrat, das auf das Titandioxid aufgebracht wird, ist Fe₂O₃, Fe₃O₄, FeOOH, Cr₂O₃, CuO, Ce₂O₃, Al₂O₃, SiO₂, BiVO₄, NiTiO₃, CoTiO₃ sowie antimondotiertes, fluordotiertes oder indiumdotiertes Zinnoxid.

In einer besonderen Ausführungsform des erfindungsgemäßen Pigmentes ist auf der 2. Schicht aus einem anderen Metalloxid oder Metalloxidhydrat noch eine 3. Schicht aus einem weiteren Metalloxid oder Metalloxidhydrat vorhanden. Dieses weitere Metalloxid oder Metalloxidhydrat ist Aluminiumoxid oder Aluminiumoxidhydrat, Siliziumdioxid oder Siliziumdioxidhydrat, Fe₂O₃, Fe₃O₄, FeOOH, ZrO₂, Cr₂O₃ sowie antimondotiertes, fluordotiertes oder indiumdotiertes Zinnoxid.

Die wäßrige Lösung einer thermisch hydrolysierbaren Titanverbindung für die Herstellung der Titandioxidplättchen auf dem endlosen Band ist bevorzugt eine wäßrige Titantetrachloridlösung.

Die Konzentration des Titansalzes in diesen Lösungen beträgt 7 bis 30 Gew.-%, vorzugsweise 8 bis 15 Gew.-%.

Weiterhin wird diese Aufgabe gemäß der Erfindung gelöst durch ein Verfahren zur Herstellung der erfindungsgemäßen Pigmente, indem
- eine wäßrige Lösung einer thermisch hydrolysierbaren Titanverbindung als dünner Film auf ein endloses Band aufgebracht wird,
- der flüssige Film durch Trocknung verfestigt wird und dabei das Titandioxid durch eine chemische Reaktion aus dem Precursor entwickelt wird,
- die entstandene Schicht anschließend vom Band abgelöst und gewaschen wird,
- die erhaltenen Titandioxidplättchen ohne Zwischentrocknung in Wasser suspendiert und mit einem anderen Metalloxid oder Metalloxidhydrat und gegebenenfalls mit weiteren Metalloxiden oder Metalloxidhydraten beschichtet werden,
- die beschichteten Titandioxidplättchen aus der wäßrigen Suspension abgetrennt, getrocknet und gegebenenfalls kalziniert werden.

Weiterhin kann die Beschichtung der Titandioxidplättchen nach Zwischentrocknung mit Metalloxiden bzw. Metalloxidhydraten auch z.B. in einem Wirbelbettreaktor durch Gasphasenbeschichtung erfolgen, wobei z.B. die in EP 0,045,851 und EP 0,106,235 zur Herstellung von Perlglanzpigmenten vorgeschlagenen Verfahren entsprechend angewendet werden können.

Gegenstand der Erfindung ist außerdem die Verwendung der erfindungsgemäßen Pigmente zur Pigmentierung von Lacken, Druckfarben, Kunststoffen, Kosmetika und Glasuren für Keramiken und Gläser.

Hierfür können sie auch als Mischungen mit handelsüblichen Pigmenten, beispielsweise anorganischen und organischen Absorptionspigmenten, Metalleffektplgmenten und LCP-Pigmenten, eingesetzt werden.

Die erfindungsgemäßen Pigmente basieren auf plättchenförmigem Titandioxid. Diese Plättchen haben eine Dicke zwischen 10 nm und 500 nm, vorzugsweise zwischen 40 und 150 nm. Die Ausdehnung in die beiden anderen Dimensionen beträgt zwischen 2 und 200 µm und insbesondere zwischen 5 und 50 µm.

Die Schicht eines anderen Metalloxids, die auf die Titandioxidplättchen aufgebracht wird, besitzt eine Dicke von 5 bis 300 nm, vorzugsweise zwischen 5 und 150 nm.

Die Herstellung der erfindungsgemäßen Pigmente erfolgt in einem dreistufigen Verfahren. In der ersten Stufe werden plättchenförmige Titandioxidpartikel mit Hilfe eines endlosen Bandes hergestellt.

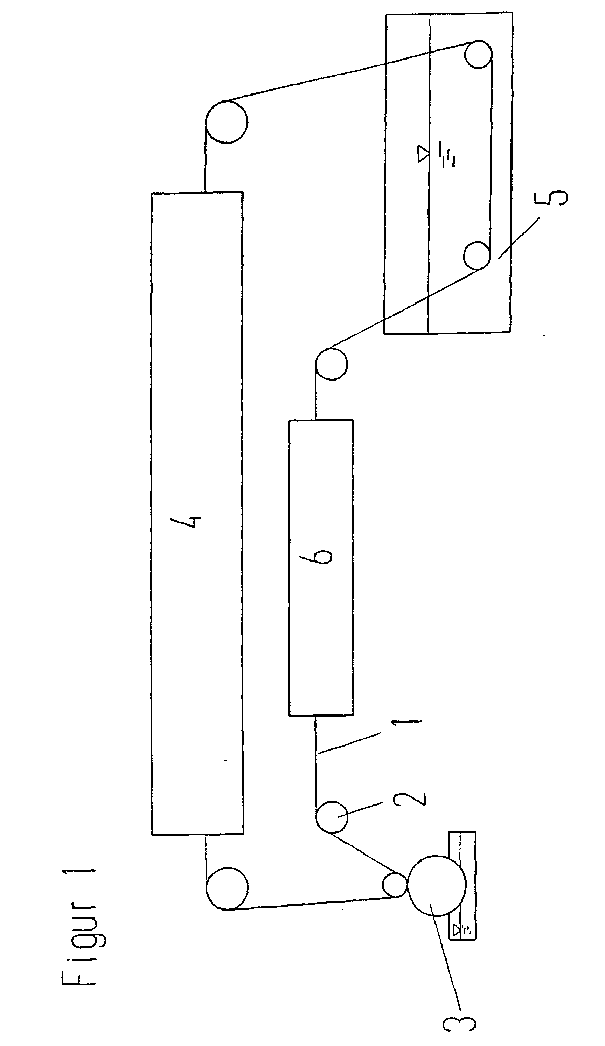

Zunächst soll an Hand von Figur 1 das Bandverfahren erläutert werden.

Das endlose Band 1, welches über ein Rollensystem 2 geführt wird, durchläuft ein Auftragswerk 3, wo es mit einem dünnen Film des Precursors beschichtet wird. Als geeignete Auftragswerke können Walzenauftragswerke sowie Fließer eingesetzt werden. Die Bandgeschwindigkeit liegt zwischen 2 und 400 m/min, vorzugsweise 5 - 200 m/min.

Um eine gleichmäßige Benetzung des Kunststoffbandes zu erzielen, ist es zweckmäßig, der Beschichtungslösung ein handelsübliches Netzmittel zuzusetzen oder die Bandoberfläche durch Beflammung, Koronabehandlung oder Ionisation zu aktivieren.

Anschließend durchläuft das beschichtete Band eine Trockenstrecke 4 in der die Schicht bei Temperaturen zwischen 30 und 200 °C getrocknet wird. Als Trockner können zum Beispiel handelsübliche Infrarot-, Umluftdüsen- und UV-Trockner eingesetzt werden.

Nach dem Passieren der Trockenstrecke wird das Band durch die Ablösebäder 5 mit einem geeignetem Ablösemedium, zum Beispiel vollentsalztem Wasser geführt, wo die getrocknete Schicht vom Band entfernt wird. Der Ablösevorgang wird hierbei durch zusätzliche Vorrichtungen, zum Beispiel Düsen, Bürsten oder Ultraschall, unterstützt.

In einem Nachtrockner 6 wird das Band vor der erneuten Beschichtung getrocknet.

Das endlose Band sollte aus einem chemisch und thermisch beständigem Kunststoff sein, um eine ausreichende Standzeit und hohe Trocknungstemperaturen zu gewährleisten. Hierfür sind Materialien wie Polyethylenterephthalat (PET) oder andere Polyester und Polyacrylate geeignet.

Die Folienbreite liegt typischerweise zwischen einigen Zentimetern bis zu mehreren Metern. Die Dicke beträgt zwischen 10 µm bis zu einigen mm, wobei diese beiden Parameter im Hinblick auf die jeweiligen Anforderungen optimiert werden.

Weitere Einzelheiten zu kontinuierlichen Bandverfahren sind aus US 3 138 475, EP 0 240 952 und WO 93/08 237 bekannt.

Die vom Band abgelösten Titandioxidplättchen werden in einer zweiten Verfahrensstufe ohne vorherige Zwischentrocknung mit einem anderen Metalloxid oder Metalloxidhydrat nach bekanntem Verfahren beschichtet.

Bei der Beschichtung mit Hämatit (Fe₂O₃) kann sowohl von Eisen(III)-salzen ausgegangen werden, wie es zum Beispiel in US 3 987 828 und US 3 087 829 beschrieben ist, als auch von Eisen(II)-salzen, wie in US 3 874 890 beschrieben, wobei der zunächst gebildete Überzug von Eisen(II)-hydroxid zum Eisen(III)-oxidhydrat oxidiert wird. Bevorzugt wird von Eisen(III)-salzen ausgegangen. Hierzu wird einer wäßrigen Suspension der Titandioxidplättchen bei einer Temperatur von 60 bis 90 °C und bei einem pH-Wert von 2,5 bis 4,5 eine Eisen(III)-chloridlösung zudosiert. Der pH-Wert wird durch gleichzeitige Dosierung von 32 %iger Natronlauge konstant gehalten. Nach Aufarbeitung und Trocknung bei 110 °C erhält man ein rotes Pigment.

Die Beschichtung mit Magnetit (Fe₃O₄) erfolgt durch Hydrolyse einer Eisen(II)-salzlösung, beispielsweise Eisen(II)-sulfat, bei einem pH-Wert von 8,0 in Gegenwart von Kaliumnitrat. Die genauen Fällungsbeispiele sind in EP 0 659 843 beschrieben. Man erhält ein schwarzes Pigment nach Aufarbeitung und Trocknung bei 100 °C.

Bevorzugt ist auch eine Beschichtung der Titandioxidplättchen mit gelbem FeO(OH) in der Goethitmodifikatian. Dabei wird zu einer Suspension der Titandioxidplättchen in einer Stickstoffatmosphäre bei 70 °C eine wäßrige FeSO₄-Lösung zudosiert. Anschließend wird mit einer 10 %igen Na₂CO₃-Lösung der pH-Wert auf 4 eingestellt und Sauerstoff in die Suspension eingeleitet. Nach Aufarbeitung und Trocknung bei 110 °C erhält man ein gelbes Pigment. Bezüglich der genauen Fällungsbedingungen wird auf EP 0 659 843 verwiesen.

Für eine bessere Haftung der Eisenoxidschichten auf den Titandioxidplättchen ist es zweckmäßig, zuerst eine Zinnoxidschicht aufzubringen. Dabei wird zu der auf einen pH-Wert von 1,8 eingestellten Suspension der Titandioxidplättchen eine mit Salzsäure versetzte SnCl₄-Lösung zudosiert und der pH-Wert durch gleichzeitige Dosierung von 32 %iger Natronlauge konstant gehalten. Nach dem Auffällen der Zinndioxydhydratschicht kann dann unmittelbar die Beschichtung mit einem Eisenoxidhydrat angeschlossen werden.

Ein anderes Metalloxid, das bevorzugt auf die Titandioxidplättchen aufgefällt wird, ist Chromoxid.

Die Abscheidung kann leicht durch die thermische Hydrolyse, die bei der Verflüchtigung von Ammoniak aus einer wäßrigen Lösung eines Hexammin-Chrom(III)-Derivates eintritt, oder durch thermische Hydrolyse einer Chromsalzlösung, die mit Borax gepuffert ist, bewirkt werden. Die Beschichtung mit Chromoxid ist in US 3 087 828 und 3 087 829 beschrieben.

Die erfindungsgemäßen Pigmente müssen nicht in jedem Falle kalziniert werden. Für bestimmte Anwendungen genügt eine Trocknung bei Temperaturen von 110°C. Wird das Pigment geglüht, werden Temperaturen zwischen 400 °C und 1000 °C eingestellt, wobei der bevorzugte Bereich zwischen 400 °C und 700 °C liegt.

Das erfindungsgemäße Pigment kann noch zusätzlich mit schwerlöslichen, fest haftenden anorganischen oder organischen Farbmitteln beschichtet werden. Bevorzugt werden Farblacke und insbesondere Aluminiumfarblacke verwendet. Dazu wird eine Aluminiumhydroxidschicht aufgefällt, die in einem zweiten Schritt mit einem Farblack verlackt wird. Das Verfahren ist in DE 24 29 762 und DE 29 28 287 näher beschrieben.

Bevorzugt ist auch eine zusätzliche Beschichtung mit Komplexsalzpigmenten, insbesondere Cyanoferratkomplexen, wie zum Beispiel Berliner Blau und Turnbulls Blau, wie sie in EP 0 141 173 und DE 23 13 332 beschrieben ist.

Das erfindungsgemäße Pigment kann auch mit organischen Farbstoffen und insbesondere mit Phthalocyanin- oder Metallphthalocyanin- und/oder Indanthrenfarbstoffen nach DE 40 09 567 beschichtet werden. Dazu wird eine Suspension des Pigmentes in einer Lösung des Farbstoffes hergestellt und diese dann mit einem Lösungsmittel zusammengebracht, in welchem der Farbstoff schwer löslich oder unlöslich ist.

Weiterhin können auch Metallchalkogenide bzw. Metallchalkogenidhydrate und Ruß für eine zusätzliche Beschichtung eingesetzt werden.

Es ist weiterhin möglich, die Pigmente einer Nachbeschichtung oder Nachbehandlung zu unterziehen, die die Licht-, Wetter- und chemische Stabilität weiter erhöht, oder die Handhabung des Pigments, insbesondere die Einarbeitung in unterschiedliche Medien, erleichtert. Als Nachbeschichtungen kommen beispielsweise die in den DE-PS 22 15 191, DE-OS 31 51 354, DE-OS 32 35 017 oder DE-OS 33 34 598 beschriebenen Verfahren in Frage. Aufgrund der bereits ohne diese zusätzlichen Maßnahmen sehr guten Eigenschaften der erfindungsgemäßen Pigmente machen diese ggf. noch aufgebrachten Stoffe nur etwa 0 bis 5, insbesondere etwa 0 bis 3 Gew.% des gesamten Pigments aus.

Die erfindungsgemäßen Pigmente stellen bezüglich der Dicke den Idealzustand dar, der bei Perlglanzpigmenten maximal erreichbar ist, da sie nur aus optisch funktionellen Schichten bestehen und ein sonst übliches Trägermaterial, wie beispielsweise Glimmer oder Glasplättchen, das nicht zum optischen Effekt beiträgt, fehlt. Bedingt durch die Dicke des Glimmers besitzen Glimmerpigmente eine bis um den Faktor 25 größere Dicke bei gleicher Dicke der funktionellen Schichten. Bezüglich der technischen Anwendungen ergeben sich hieraus intrinsische Vorteile, die von keinem anderen konventionellen Perlglanzpigment erreicht werden können. Beispielsweise können Lackschichten dünner ausgeführt werden und die notwendige Pigmentmenge kann reduziert werden, weil aufgrund des Fehlens des "Füllstoffs" Trägermaterial die Pigmente optisch aktiver sind.

Das nachfolgende Beispiel soll die Erfindung erläutern, ohne sie zu begrenzen.

### Beispiel 1

Ein umlaufendes Band aus Polyethylenterephthalat (Breite: 0,3m, Geschwindigkeit: 20 m/min) wird über eine Auftragswalze im Gegenlauf mit einer 20%iger Titantetrachloridlösung beschichtet. Die Beschichtungslösung enthält 0,3 Gew.-% Tensid (DISPERSE-AYD W-28, Hersteller: DANIEL PRODUCTS COMPANY). Der auf dem Band befindliche wäßrige Film wird in einer Trocknungsstrecke durch Beaufschlagung mit Heißluft von 70 °C getrocknet und die gebildete Schicht in einem mit vollentsalztem Wasser gefüllten Ablösebecken vom Band abgelöst. Die plättchenförmigen Titandioxidpartikel werden filtriert und mit vollentsalztem Wasser gewaschen. Die silbrig glänzenden Plättchen haben eine Schichtdicke von 100 ± 10 nm. Zur Beschichtung mit einem anderen Metalloxid werden sie in vollentsalztem Wasser redispergiert.

2 I der Dispersion (Trockensubstanzgehalt: 15 g TiO₂) werden auf 75 °C erhitzt und mit verdünnter Salzsäure auf einen pH-Wert von 1,8 eingestellt.

4,3 g SnCl₄ · 5 H₂O werden in 29 ml HCI gelöst, mit destilliertem Wasser auf 290 ml aufgefüllt und 10 min gerührt.

Die SnCl₄-Lösung wird mit 3 ml/min zur TiO₂-Suspension zugegeben und dabei der pH 1,8 mit 32%iger NaOH-Lösung konstant gehalten. Nach der SnO₂-Belegung wird die Suspension 15 min bei konstanter Temperatur und konstantem pH-Wert nachgerührt.

Anschließend wird der pH-Wert mit 32 %iger NaOH-Lösung auf 3,0 eingestellt und eine wäßrige 8 %ige Eisen(III)-chloridlösung mit einer Geschwindigkeit von 3 ml/min zudosiert, wobei der pH-Wert durch gleichzeitige Zugabe der NaOH-Lösung konstant gehalten wird. Die FeCl₃-Zugabe wird fortgesetzt bis die gewünschte Interferenzfarbe erreicht ist. Das erhaltene Pigment wird abfiltriert, mit vollentsalztem Wasser gewaschen und bei 110 °C 12 Stunden getrocknet. Das Pigment besitzt die gewünschte Interferenzfarbe und eine rote Körperfarbe.

## Patentansprüche

1. Farbiges Perlglanzpigment, bestehend aus einem Kern aus plättchenförmigem Titandioxid und einer oder mehrerer Schichten aus anderen Metalloxiden bzw. Metalloxidhydraten, erhältlich durch Verfestigung einer wäßrigen Lösung einer thermisch hydrolisierbaren Titanverbindung auf einem endlosen Band, Ablösung der entstandenen Schicht, Beschichtung der erhaltenen Titandioxidplättchen ohne Zwischentrocknung im Naßverfahren mit einem oder mehreren anderen Metalloxiden bzw. Metalloxidhydraten, Abtrennung, Trocknung und gegebenenfalls Kalzination des erhaltenen Materials.

2. Perlglanzpigment nach Anspruch 1, **dadurch gekennzeichnet, daß** das andere Metalloxid Fe₂O₃, Fe₃O₄, FeOOH oder Cr₂O₃ ist.

3. Verfahren zur Herstellung des Perlglanzpigmentes nach den Ansprüchen 1 bis 2, **dadurch gekennzeichnet, daß**
- eine wäßrige Lösung einer thermisch hydrolysierbaren Titanverbindung als dünner Film auf ein endloses Band aufgebracht wird,
- der flüssige Film durch Trocknung verfestigt wird und dabei das Titandioxid durch eine chemische Reaktion aus der Lösung entwickelt wird,
- die entstandene Schicht anschließend vom Band abgelöst und gewaschen wird,
- die erhaltenen Titandioxidplätten ohne Zwischentrocknung in Wasser suspendiert und mit einem oder mehreren Metalloxiden oder Metalloxidhydraten beschichtet werden,

4. Verfahren nach Anspruch 3, **dadurch gekennzeichnet, daß** die wäßrige Lösung einer thermisch hydrolisierbaren Titanverbindung eine wäßrige Titantetrachloridlösung ist.

5. Verfahren nach den Ansprüchen 3 und 4, **dadurch gekennzeichnet, daß** als andere Metalloxide Fe₂O₃, Fe₃O₄, FeOOH und Cr₂O₃ eingesetzt werden.

6. Verfahren nach mindestens einem der Ansprüche 3 bis 5, **dadurch gekennzeichnet, daß** das Pigment bei Temperaturen von 110 bis 150 °C getrocknet wird.

7. Verfahren nach mindestens einem der Ansprüche 3 bis 6, **dadurch gekennzeichnet, daß** das Pigment bei Temperaturen von 400 bis 700 °C kalziniert wird.

8. Verfahren nach mindestens einem der Ansprüche 3 bis 7, **dadurch gekennzeichnet, daß** weitere Metalloxide in einem Wirbelbettreaktor durch CVD auf die Titandioxidplättchen aufgebracht werden.

9. Verwendung der Pigmente gemäß den Ansprüchen 1 und 2 zur Pigmentierung von Lacken, Druckfarben, Kunststoffen, Kosmetika und Glasuren für Keramiken und Gläser.

10. Verwendung nach Anspruch 9, **dadurch gekennzeichnet, daß** die Pigmente als Mischungen mit handelsüblichen Pigmenten eingesetzt werden.

11. Lacke, Druckfarben, Kunststoffe und Kosmetika, welche mit einem Pigment nach den Ansprüchen 1 und 2 pigmentiert sind.

## Claims

1. Coloured pearl lustre pigment, consisting of a core of plateletlike titanium dioxide and one or more layers of other metal oxides or metal oxide hydrates obtainable by solidifying an aqueous solution of a thermally hydrolysable titanium compound on a continuous belt, detaching the resulting layer, coating the resulting titanium dioxide platelets, without drying in between, with one or more other metal oxides or metal oxide hydrates by a wet method, separating, drying and, if desired, calcining the material obtained.

2. Pearl lustre pigment according to Claim 1, **characterized in that** the other metal oxide is Fe₂O₃, Fe₃O₄, FeOOH or Cr₂O₃.

3. Process for the preparation of the pearl lustre pigment according to Claims 1 to 2, **characterized in that**
- an aqueous solution of a thermally hydrolysable titanium compound is applied as a thin film to a continuous belt,
- the liquid film is solidified by drying, during the course of which the titanium dioxide is developed from the solution by means of a chemical reaction,
- the resulting layer is subsequently detached from the belt and washed,
- the titanium dioxide platelets obtained, without drying in between, are suspended in water and coated with one or more metal oxides or metal oxide hydrates,
- the coated titanium dioxide platelets are separated out from the aqueous suspension, dried and, if desired, calcined.

4. Process according to Claim 3, **characterized in that** the aqueous solution of a thermally hydrolysable titanium compound is an aqueous titanium tetrachloride solution.

5. Process according to Claims 3 and 4, **characterized in that** the other metal oxides employed are Fe₂O₃, Fe₃O₄, FeOOH and Cr₂O₃.

6. Process according to at least one of Claims 3 to 5, **characterized in that** the pigment is dried at temperatures from 110 to 150°C.

7. Process according to at least one of Claims 3 to 6, **characterized in that** the pigment is calcined at temperatures from 400 to 700°C.

8. Process according to at least one of Claims 3 to 7, **characterized in that** further metal oxides are applied to the titanium dioxide platelets in a fluidized-bed reactor by CVD.

9. Use of the pigments according to Claims 1 and 2 for pigmenting paints, printing inks, plastics, cosmetics and glazes for ceramics and glass.

10. Use according to Claim 9, **characterized in that** the pigments are employed as mixtures with commercially available pigments.

11. Paints, printing inks, plastics and cosmetics which are pigmented with a pigment according to Claims 1 and 2.

## Revendications

1. Pigment de lustre nacré coloré, constitué par un noyau en dioxyde de titane sous forme de plaquette et une ou plusieurs couches d'autres oxydes métalliques, respectivement hydrates d'oxydes métalliques, pouvant être obtenu par stabilisation d'une solution aqueuse d'une combinaison titane thermiquement hydrolysable sur une bande sans fin, décollement de la couche ainsi formée, revêtement des plaquettes d'oxyde de titane obtenues en l'état humide sans séchage intermédiaire, avec un ou plusieurs autres oxydes métalliques, respectivement hydrates d'oxydes métalliques, séparation, séchage et, le cas échéant, calcination du matériau obtenu.

2. Pigment de lustre nacré coloré selon la revendication 1, **caractérisé en ce que** l'autre oxyde métallique est Fe₂O₃, Fe₃O₄, FeOOH ou Cr₂O₃.

3. Procédé pour la fabrication du pigment de lustre nacré coloré selon les revendications 1 à 2, **caractérisé en ce que**
- une solution aqueuse d'une combinaison titane thermiquement hydrolysable est appliquée en tant que film mince sur une bande sans fin;
- le film liquide est solidifié par séchage et qu'en ce faisant, l'oxyde de titane est formé par réaction chimique à partir de la solution ;
- la couche formée est décollée de la bande et lavée ;
- les plaquettes de dioxyde de titane obtenues sont fluidisées, sans séchage intermédiaire, dans de l'eau et recouvertes avec un ou plusieurs oxydes métalliques, ou des hydrates d'oxydes métalliques ;
- les plaquettes de dioxyde de titane revêtues sont séparées de la suspension aqueuse, séchées et, le cas échéant, calcinées.

4. Procédé selon la revendication 3, **caractérisé en ce que** la solution aqueuse d'une combinaison titane thermiquement hydrolysable est une solution aqueuse de tétrachlorure de titane.

5. Procédé selon les revendications 3 et 4, **caractérisé en ce que** les autres oxydes métalliques mis en oeuvre sont Fe₂O₃, Fe₃O₄, FeOOH et Cr₂O₃.

6. Procédé selon au moins l'une des revendications 3 à 5, **caractérisé en ce que** le pigment est séché à des températures allant de 110 à 150 °C.

7. Procédé selon au moins l'une des revendications 3 à 6, **caractérisé en ce que** le pigment est calciné à des températures allant de 400 à 700 °C.

8. Procédé selon au moins l'une des revendications 3 à 7, **caractérisé en ce que** d'autres oxydes métalliques sont appliqués sur les plaquettes d'oxyde de titane par procédé CVD dans un réacteur à lit fluidisé.

9. Utilisation des pigments selon les revendications 1 et 2 pour la pigmentation de laques, encres d'imprimerie, matières plastiques, cosmétiques et émaux pour la céramique et le verre.

10. Utilisation selon la revendication 9, **caractérisée en ce que** les pigments sont mis en oeuvre en tant que mélanges avec des pigments du commerce.

11. Laques, encres d'imprimerie, matières plastiques et cosmétiques pigmentées avec un pigment selon les revendications 1 et 2.
